(19) **Europäisches Patentamt**

**European Patent Office**

**Office européen des brevets**

(11) **EP 0 895 976 B1**

(12) **EUROPEAN PATENT SPECIFICATION**

(45) Date of publication and mention
of the grant of the patent:
**29.10.2003 Bulletin 2003/44**

(51) Int Cl.⁷: **C07C 2/66**, C07C 15/107

(21) Application number: **98304551.9**

(22) Date of filing: **09.06.1998**

(54) **Process for the alkylation of heavy mono-alkylates of single-ring aromatic hydrocarbons**

Verfahren zur Alkylierung von schweren Monoalkylaten von monozyklischen aromatischen Kohlenwasserstoffen

Procédé pour l'alkylation de monoalkylates lourdes d'hydrocarbures aromatiques monocycliques

(84) Designated Contracting States:
**DE FR GB NL**

(30) Priority: **06.08.1997 US 907334**

(43) Date of publication of application:
**10.02.1999 Bulletin 1999/06**

(73) Proprietor: **Chevron Oronite Company LLC
San Ramon, CA 94583-4289 (US)**

(72) Inventors:
• **Campbell, Curt B.
Hercules, CA 94547 (US)**

• **Triconnet, Thierry
76430 Saint Romain de Colboso (FR)**

(74) Representative: **Nash, David Allan
Haseltine Lake & Co.,
Imperial House, 15-19 Kingsway
London WC2B 6UD (GB)**

(56) References cited:
**US-A- 4 148 834          US-A- 5 146 026**

**Description**

The alkylation of aromatics with a variety of Lewis or Brönsted acid catalysts is well known. Typical commercial catalysts include phosphoric acid/kieselguhr, aluminum chloride, and hydrogen fluoride. Alkylation with lower molecular weight olefins, such as propylene, can be carried out in the liquid or vapor phase. For alkylations with higher olefins, such as $C_{16+}$ olefins, the alkylations are done in the liquid phase, usually in the presence of hydrogen fluoride. Alkylations of benzene with higher olefins is especially difficult, and requires hydrogen fluoride treatment. Such a process is disclosed by Himes in U.S. Patent No. 4,503,277, entitled "HF Regeneration in Aromatic Hydrocarbon Alkylation Process," which is hereby incorporated by reference in its entirety for all purposes. However, hydrogen fluoride is not environmentally attractive.

Alkylates derived from heavy olefin feeds (olefins having at least sixteen carbon atoms) used in the manufacturing of lubrication oil sulfonates and as carrier fluids typically contain some amount (5 to 15%) of heavy-alkylate species. For certain functions, it is desirable to maximize this heavy-alkylate content for performance reasons, however increasing the heavy-alkylate content above 20 weight percent is not a trivial task, because the conditions that favor di-alkylation usually favors the formation of large amounts of very high molecular weight olefin oligomers.

U.S. Patent No. 4,148,834, entitled "Preparation of Synthetic Hydraulic Lubricants," discloses the production of dialkylaromatic hydrocarbons from aromatic hydrocarbons by first reacting from one to fifteen moles of aromatic hydrocarbon with one mole of a $C_6$-$C_{18}$ linear monoolefin at a temperature of from 5 to 100° C in the presence of from one to thirty moles HF per mole of aromatic hydrocarbon, then recovering the monoalkylaromatic from the reaction mixture and then reacting from one to ten moles of the monoalkylaromatic with one mole of linear $C_6$-$C_{18}$ mono-olefin at a temperature of from 60° to 90°C in the present of from 2 to 10 weight %, based on the olefin, of $AlCl_3$ or $AlBr_3$, and recovering the dialkylaromatic hydrocarbons from the reaction mixture.

U.S. Patent No. 5,146,026 discloses a process for the alkylation of aromatic hydrocarbons with a $C_2$ to $C_{20}$ mono-olefin, in the present of a zeolite or a clay as a catalyst.

## SUMMARY OF THE INVENTION

According to the present invention, there is provided a process for increasing the heavy-alkylate content of a heavy mono-alkylated single-ring aromatic hydrocarbon to at least 20 weight percent, wherein the alkyl group of the mono-alkylated single-ring aromatic hydrocarbon has at least 20 carbon atoms and wherein the term "heavy-alkylate" means chemical species having a molecular weight higher than that of the mono-alkylated single-ring aromatic hydrocarbon, which process comprises:

> reacting the heavy mono-alkylated single-ring aromatic hydrocarbon with an olefin having from 6 to 14 carbon atoms at a pressure of up to 10 bars and a temperature of from 100° to 225°C in the presence of an acidic catalyst selected from molecular sieves and clays.

Thus, the present invention provides a process for increasing the heavy-alkylate content of heavy mono-alkylates to at least 20 weight percent, preferably at least 50 weight percent, heavy-alkylate. The process comprises reacting a heavy mono-alkylated single-ring aromatic hydrocarbon with an olefin having from 6 to 14 carbon atoms at a pressure of up to about 10 bars and a temperature of from 100° to 225°C in the presence of an acidic catalyst. The acidic catalyst can be a clay or a molecular sieve (such as a natural or synthetic zeolite).

Preferably, the single-ring aromatic hydrocarbon that is alkylated is benzene, toluene, cumene, xylene, or a mixture thereof. Most preferably, the single-ring aromatic hydrocarbon is benzene.

Preferably, the alkyl group of the mono-alkylated single-ring aromatic hydrocarbon has from 20 to 28 carbon atoms, and the mono-alkylate content of the mono-alkylated single-ring aromatic hydrocarbon is at least 80 weight percent.

## DETAILED DESCRIPTION OF THE INVENTION

In its broadest aspect, the present invention involves a process for producing an alkylated single-ring aromatic hydrocarbon having a heavy-alkylate content of at least 20 weight percent, preferably at least 50 weight percent.

By "heavy-alkylate", we mean the amount of total alkylate that is comprised of those chemical species present with molecular weights higher than that of the mono-alkylate. These chemical species present with molecular weights higher than that of the mono-alkylate may be composed of, but are not limited to:

> (a) mono-alkylated aromatics of oligomerized olefins,
> (b) di-alkylated aromatic species,

(c) tri-alkylated aromatic species, and

(d) oligomerized olefins species.

## HEAVY MONO-ALKYLATED SINGLE-RING AROMATIC HYDROCARBON

In the present invention, a "heavy mono-alkylated single-ring aromatic hydrocarbon" is reacted with an olefin under certain conditions to produce an alkylate with a high heavy-alkylate content.

By the phrase "heavy mono-alkylated single-ring aromatic hydrocarbon," we mean an aromatic hydrocarbon containing a single aromatic ring and having attached to that single aromatic ring a single long-chain alkyl group having at least twenty carbon atoms. Preferably, the long-chain alkyl group is derived from normal alpha olefins, but it can contain some degree of branching. Preferably, the long-chain alkyl group has from 20 to 28 carbon atoms.

The single-ring aromatic hydrocarbon that is alkylated is preferably benzene, toluene, cumene, xylene, or mixtures thereof. The "alkyl group" refers to the long-chained alkyl group attached by alkylation, and not to any methyl group or groups. We do not consider methyl groups to be long-chain.

Preferably, the mono-alkylate content of the mono-alkylated single-ring aromatic hydrocarbon is at least 80 weight percent. By "mono-alkylate content," we mean the amount of alkylated single-ring aromatic hydrocarbon that only has one long-chain alkyl group. The remainder could consist of minor amounts of di-alkylated single-ring aromatic hydrocarbons, tri-alkylated single-ring aromatic hydrocarbons, oligomerized olefins, and alkylates formed from oligomerized olefins.

The alkylated single-ring aromatic hydrocarbon can be formed by any conventional process, such as processes similar to those disclosed by Resh in U.S. Patent No. 4,691,098 entitled "Process for Production of Alkyl Aromatics," and disclosed by Kocal in U.S. Patent No. 5,334,793 entitled "Increasing Catalyst Life and Improving Product Linearity in the Alkylation of Aromatics with Linear Olefins." Both U.S. Patent Nos. 4,691,098 and 5,334,793 are hereby incorporated by reference in their entirety for all purposes.

## OLEFIN

In the present invention, a heavy mono-alkylated single-ring aromatic hydrocarbon is reacted with an olefin having from 6 to 14 carbon atoms. Preferably, the olefin is a normal alpha olefin, but the olefin can contain some degree of branching.

## CATALYST

The catalyst of the present invention is a solid, acidic catalyst, such as a molecular sieve or clay. Preferred molecular sieves include natural and synthetic zeolites.

Preferably, the acidic catalyst comprises the acid forms of an acidic clay, or an acidic molecular sieve (such as a zeolite) having a pore size of at least 6.0 Angstroms. Such zeolites include zeolite Y, beta, SSZ-25, SSZ-26, and SSZ-33. Other possible catalysts include L zeolite, mordenite, boggsite, cloverite, VPI-5, MCM-41, MCM-36, SAPO-8, SAPO-5, MAPO-36, SAPO-40, SAPO-41, MAPSO-46, CoAPO-50, hexagonal faujasite (EMC-2), gmelinite, mazzite (omega zeolite), offretite, ZSM-18, and ZSM-12. These catalysts are discussed in Rosemarie Szostak's *Handbook of Molecular Sieves* (New York, Van Nostrand Reinhold, 1992). The catalyst is activated prior to use. Preferably, the activated catalyst is used without exposure to atmospheric water. Most preferably, the acidic catalyst is an acidic clay.

Useful acidic clays may be derived from naturally occurring or synthetic materials. One skilled in the art would realize that there are a number of such clays that are known to be alkylation catalysts. Examples of such acidic clays include montmorillonite, laponite, and saponite. Pillared clays may also be used as catalysts.

## PROCESS CONDITIONS

The alkylation reaction is typically carried out with a heavy mono-alkylated single-ring aromatic hydrocarbon and an olefin in molar ratios of about from 1:15 to 25:1. The process is typically carried out at a pressure of up to about 10 bars and a temperature of about from 100° to 225° C in the presence of an acidic catalyst. The process can be operated using catalysts in either a batch or fixed bed reactor. As the olefin has a low boiling point, the process is preferably carried out in the liquid phase. The alkylation process may be carried out in batch or continuous mode. In the batch mode a typical method is to use a stirred autoclave or glass flask which may be heated to the desired reaction temperature. A continuous process is most efficiently carried out in a fixed bed process. Space rates in a fixed bed process can be in the range of about from 0.01 to 10 or more weight hourly space velocity (WHSV).

In a fixed bed process the catalyst is charged to the reactor and activated or dried at a temperature of at least 100°C under flowing inert, dry gas. After activation, the catalyst is cooled to ambient temperature and a flow of the heavy mono-alkylated single-ring aromatic hydrocarbon is introduced. Pressure is increased by means of a back pressure valve so that the pressure is above the bubble point pressure of the aromatic species at the desired reaction temperature. After pressurizing the system to the desired pressure, the temperature is increased to the desired reaction temperature. A flow of the olefin is then mixed with the heavy mono-alkylated single-ring aromatic hydrocarbon and is allowed to flow over the catalyst. The reactor effluent containing dialkylate product and excess aromatic is collected. Excess olefin is then removed by distillation, stripping, evaporation under vacuum, or other means known to those skilled in the art.

## EXAMPLES

The invention will be further illustrated by following examples, which set forth particularly advantageous method embodiments. The Examples are provided to illustrate the present invention. Unless stated otherwise, all percentages in the Examples are weight percentages.

### EXAMPLE 1

A commercial acidic zeolite Y extrudate catalyst available from Aldrich Chemical Company (1.85 grams) was weighed into a 30 cc glass serum bottle. The bottle was then placed in an oven at 100° C in air for approximately 17 hours. The bottle was then removed from the oven and immediately sealed with a TEFLON rubber faced septum using a crimpon tool. After the bottle had cooled to room temperature, 2 ml of a $C_{20-24}$ NAO alkyl benzene alkylate (consisting of 88 % mono-alkylate and 12 % heavy alkylate by Supercritical Fluid Chromatography (SFC)) and 5 ml of 1-decene were added to the vial via syringe. The bottle was then placed in an oil bath maintained at between 145° C and 155° C. After 24 hours, the bottle was removed and allowed to cool to room temperature and then the bottle was opened and the contents were gravity filtered through filter paper to afford a liquid product. Analysis of this liquid by SFC showed the following composition: 31 % 1-decene dimer; 53 % mono-alkylate and 16 % heavy-alkylate. The ratio of mono-alkylate to heavy alkylate in this product is 77:23. In other words, the heavy-alkylate content of the total alkylate product was 23 %.

### EXAMPLE 2

To a 30 cc glass serum bottle was added 1.85 grams of Filtrol F-24 acid clay catalyst granules (available from Englehard Corporation, Elyria, Ohio). The bottle was then placed in an oven at 100°C in air for approximately 17 hours. The bottle was then removed from the oven and immediately sealed with a TEFLON rubber faced septum using a crimpon tool. After the bottle had cooled to room temperature, 2 ml of a $C_{20-24}$ NAO alkyl benzene alkylate (consisting of 88 % mono-alkylate and 12 % heavy alkylate by SFC) and 5 ml of 1-decene were added to the vial via syringe. The bottle was then placed in an oil bath maintained at between 145° C and 155° C. After 24 hours, the bottle was removed and allowed to cool to room temperature and then the bottle was opened and the contents were gravity filtered through filter paper to afford a liquid product. Analysis of this liquid by SFC showed the following composition: 11 % 1-decene dimer; 25 % mono-alkylate and 64 % heavy alkylate. The ratio of mono-alkylate to heavy-alkylate is 15:85. In other words, the heavy-alkylate content of the total alkylate product was 85 %.

### Procedure for Determining Composition of Alkylated Heavy Alkylates by Supercritical Fluid Chromatography (SFC)

A Dionex, Lee Scientific Model 600 Supercritical Fluid Chromatograph (SFC) equipped with a 10 meter x 195 micron OD/50 micron ID, 0.25 micron film (SB-Methyl-100) capillary column, and FID detector operating at 325° C and carbon dioxide eluent was used with split injection. The following density ramp program was used (isothermal oven at 100° C):

Initial Density = 0.2 g/cc
Inject Sample
Hold five minutes
Ramp to 0.3 g/cc at 0.02 g/cc/min
Ramp to 0.5 g/cc at 0.01 g/cc/min
Ramp to 0.76 g/cc at 0.02 g/cc/min
Hold twelve minutes

For the benzene alkylates studied and under the conditions that contribute to the relative retention times (carrier gas flow, condition of the column, and other factors), the 1-decene dimer eluted between 21 and 26 minutes, the $C_{18}$ NAO benzene mono-alkylate eluted between 27 and 32,5 minutes, the $C_{18}$ NAO benzene heavy-alkylate eluted between 33 and 43 minutes, the $C_{20-24}$ NAO benzene mono-alkylate eluted between 28 and 34 minutes, the $C_{20-24}$ NAO heavy-alkylate eluted between 34.5 and 45 minutes.

The composition of the products were calculated as follows:

$$\text{Percent decene Dimer} = \frac{\text{Peak Area between 21 and 26 minutes}}{\text{Peak Area between 20 and 45 minutes}} \times 100$$

$$\text{Percent C}_{18}\text{ NAO Benzene Mono-Alkylate} = \frac{\text{Peak Area between 27 and 32.5 minutes}}{\text{Peak Area between 20 and 45 minutes}} \times 100$$

$$\text{Percent C}_{18}\text{ NAO Benzene Heavy-Alkylate} = \frac{\text{Peak Area between 33 and 43 minutes}}{\text{Peak Area between 20 and 45 minutes}} \times 100$$

$$\text{Percent C}_{20-24}\text{ NAO Benzene Mono-Alkylate} = \frac{\text{Peak Area between 28 and 34 minutes}}{\text{Peak Area between 20 and 45 minutes}} \times 100$$

$$\text{Percent C}_{20-24}\text{ NAO Benzene Heavy-Alkylate} = \frac{\text{Peak Area between 34.5 and 45 minutes}}{\text{Peak Area between 20 and 45 minutes}} \times 100$$

**Procedure for Determining the Number Average Molecular Weight (Mn), Weight Average Molecular Weight (Mw) and Heavy-Alkylate Content By Size Exclusion Chromatography (SEC)**

The chromatographic system used to determine Mn, Mw and Heavy-alkylate Content consisted of the following:

Waters model M 510 HPLC pump
Rheodyne model 71-25 injection valve fitted with a 10 μl injection loop
SEC column (stainless steel, 60 cm x 7mm ID) filled with polystyrene divinylbenzene PL Gel (10 micron size, 100 Angstrom porosity from Touzard and Matignon)
Waters model 410 differential refractomer detector
Shimadzu-Chromatopac C-R3 integrator

The mobile phase used was HPLC grade tetrahydrofuran (THF) degassed with helium and stored under helium. The chormatographic conditions used were: THF flow = 1.0 ml/min; Column temperature = 25° C, Detector Temperature 34° C, Column pressure (maximum) = 103.4 bars (1500 psi).
The column was calibrated using polystyrene standards as follows:

| Time (Minutes) | Molecular Weight |
|---|---|
| 11.905 | 834 |
| 12.438 | 582 |
| 13.087 | 386 |
| 13.336 | 358 |
| 13.468 | 330 |
| 13.934 | 274 |
| 14.234 | 246 |
| 17.110 | 92 (Toluene) |

The sample to be analyzed (0.1 gm) was dissolved in 0.02 gm toluene and 1.5 ml THF. The amount of sample injected onto the SEC column was 0.67 gm. Quantitative analysis of the chromatogram was performed by the integrator using toluene as an internal retention time calibration standard.

**COMPARATIVE EXAMPLE A**

A flow alkylation reactor operated under various conditions was used to alkylate a $C_{20-24}$ NAO benzene

alkylate (composed of 90 % mono-alkylate and 10 % heavy alkylate by SEC) with 1-octene. Following reaction, the products were distilled to remove the HF catalyst. The products were then analyzed by SEC. The different reactor conditions and products obtained are summarized in the following table.

| Sample=> | Feed | 1 | 2 | 3 | 4 | 5 |
|---|---|---|---|---|---|---|
| **REACTOR CONDITIONS** | | | | | | |
| Reactor Temperature (°C) | | 35 | 50 | 50 | 50 | 65 |
| HF/$C_8$ CMR | | 2 | 2 | 1 | 4 | 4 |
| $C_{20-24}$ Alkylate/$C_8$ CMR | | 2 | 2 | 2 | 2 | 2 |
| HF Flow (cc/min) | | 16.0 | 16.0 | 9.0 | 26.0 | 26.0 |
| $C_{20-24}$ Alkylate Flow (cc/min) | | 53.0 | 53.0 | 59.0 | 44.0 | 44.0 |
| $C_8$ Flow (cc/min) | | 8.0 | 8.0 | 9.0 | 7.0 | 7.0 |
| | | | | | | |
| **PRODUCT COMPOSITION** | | | | | | |
| Molecular Weight (Number Ave.) | 383 | 365 | 369 | 371 | 368 | 370 |
| Molecular Weight (Weight Ave.) | 408 | 401 | 404 | 404 | 402 | 403 |
| Heavy-alkylate content (wt. %) | 10.2 | 10.9 | 10.9 | 11.3 | 10.6 | 10.7 |

This comparative example shows that further alkylation using an HF catalyst does not cause a significant increase in heavy-alkylate content.

**Claims**

1. A process for increasing the heavy-alkylate content of a heavy mono-alkylated single-ring aromatic hydrocarbon to at least 20 weight percent, wherein the alkyl group of the mono-alkylated single-ring aromatic hydrocarbon has at least 20 carbon atoms and wherein the term "heavy-alkylate" means chemical species having a molecular weight higher than that of the mono-alkylated single-ring aromatic hydrocarbon, which process comprises:

   reacting the heavy mono-alkylated single-ring aromatic hydrocarbon with an olefin having from 6 to 14 carbon atoms at a pressure of up to 10 bars and a temperature of from 100° to 225°C in the presence of an acidic catalyst selected from molecular sieves and clays.

2. A process according to claim 1 wherein said molecular sieve is selected from natural zeolites and synthetic zeolites.

3. A process according to claim 1 wherein the reaction product of said process has a heavy-alkylate content of at least 50 weight percent.

4. A process according to claim 1 wherein said heavy mono-alkylated single-ring aromatic hydrocarbon is formed by the alkylation of a single-ring aromatic hydrocarbon selected from benzene, toluene, cumene, xylene, and mixtures thereof.

5. A process according to claim 4 wherein said heavy mono-alkylated single-ring aromatic hydrocarbon is formed by the alkylation of benzene.

6. A process according to claim 1 wherein the alkyl group of the mono-alkylated single-ring aromatic hydrocarbon has from 20 to 28 carbon atoms, and wherein the mono-alkylate content of the initial mono-alkylated single-ring aromatic hydrocarbon is at least 80 weight percent.

7. A process according to claim 1 wherein said acidic catalyst is an acidic clay.

8. A process according to claim 1 for increasing the heavy-alkylate content of a heavy benzene mono-alkylate to at least 50 weight percent, said process comprising reacting the heavy benzene mono-alkylate with an olefin having from 6 to 14 carbon atoms at a pressure of up to 10 bars and a temperature of from 100° to 225°C in the presence of an acidic clay catalyst, wherein the mono-alkylate content of the initial mono-alkylated benzene is at least 80 weight percent, and wherein the alkyl group of the mono-alkylated benzene has from 20 to 28 carbon atoms.

**Patentansprüche**

1.   Verfahren zum Steigern des Gehalts an schwerem Alkylat in einem schwer monoalkylierten, monozyklischen aromatischen Kohlenwasserstoff auf mindestens 20 Gew.%, wobei die Alkylgruppe des monoalkylierten, monozyklischen aromatischen Kohlenwasserstoffs mindestens 20 Kohlenstoffatome hat und der Begriff "schweres Alkylat" für eine chemische Spezies steht, deren Molekülmasse größer ist als die des monoalkylierten, monozyklischen aromatischen Kohlenwasserstoffs, umfassend:

   Umsetzen des schwer monoalkylierten, monozyklischen aromatischen Kohlenwasserstoffs mit einem Olefin, das 6 bis 14 Kohlenstoffatome besitzt, bei einem Druck von bis zu 10 bar und einer Temperatur von 100°C bis 225°C in Gegenwart eines sauren Katalysators, ausgewählt aus Molekularsieben und Tonen.

2.   Verfahren nach Anspruch 1, wobei das Molekularsieb aus natürlichen Zeolithen und synthetischen Zeolithen ausgewählt ist.

3.   Verfahren nach Anspruch 1, wobei das Reaktionsprodukt des Verfahrens einen Gehalt an schwerem Alkylat von mindestens 50 Gew.% hat.

4.   Verfahren nach Anspruch 1, wobei der schwer monoalkylierte, monozyklische aromatische Kohlenwasserstoff hergestellt wird durch Alkylierung eines monozyklischen aromatischen Kohlenwasserstoffs, ausgewählt aus Benzol, Toluol, Cumol, Xylol und deren Gemischen.

5.   Verfahren nach Anspruch 4, wobei der schwer monoalkylierte, monozyklische aromatische Kohlenwasserstoff hergestellt wird durch Alkylierung von Benzol.

6.   Verfahren nach Anspruch 1, wobei der Alkylrest des schwer monoalkylierten, monozyklischen aromatischen Kohlenwasserstoffs 20 bis 28 Kohlenstoffatome hat und der Gehalt an Monoalkylat beim monoalkylierten, monozyklischen aromatischen Ausgangskohlenwasserstoff mindestens 80 Gew.% beträgt.

7.   Verfahren nach Anspruch 1, wobei der saure Katalysator saurer Ton ist.

8.   Verfahren nach Anspruch 1 zum Erhöhen des Gehalts an schwerem Alkylat bei einem schweren Benzolmonoalkylat auf mindestens 50 Gew.%, umfassend das Umsetzen von schwerem Benzolmonoalkylat mit einem Olefin, das 6 bis 14 Kohlenstoffatome hat, bei einem Druck von bis zu 10 bar und einer Temperatur von 100°C bis 225°C in Gegenwart eines sauren Ton-Katalysators, wobei der Monoalkylatgehalt im monoalkylierten Ausgangsbenzol mindestens 80 Gew.% beträgt und der Alkylrest des monoalkylierten Benzols 20 bis 28 Kohlenstoffatome hat.

**Revendications**

1.   Procédé pour porter la teneur en alkylat lourd d'un hydrocarbure aromatique monocyclique mono-alkylé lourd à au moins 20 % en poids, dans lequel le groupe alkyle de l'hydrocarbure aromatique monocyclique mono-alkylé possède au moins 20 atomes de carbone et dans lequel l'expression "alkylat lourd" désigne une entité chimique ayant un poids moléculaire supérieur à celui de l'hydrocarbure aromatique monocyclique mono-alkylé, procédé qui comprend l'étape consistant :

   à faire réagir l'hydrocarbure aromatique monocyclique mono-alkylé lourd avec une oléfine ayant 6 à 14 atomes de carbone à une pression allant jusqu'à 10 bars et une température comprise dans l'intervalle de 100° à 225°C en présence d'un catalyseur acide choisi entre des tamis moléculaires et des argiles.

2.   Procédé suivant la revendication 1, dans lequel ledit tamis moléculaire est choisi entre des zéolites naturelles et des zéolites synthétiques.

3.   Procédé suivant la revendication 1, dans lequel le produit de réaction dudit procédé a une teneur en alkylat lourd d'au moins 50 % en poids.

**4.** Procédé suivant la revendication 1, dans lequel ledit hydrocarbure aromatique monocyclique mono-alkylé lourd est formé par alkylation d'un hydrocarbure aromatique monocyclique choisi entre le benzène, le toluène, le cumène, le xylène et leurs mélanges.

**5.** Procédé suivant la revendication 4, dans lequel ledit hydrocarbure aromatique monocyclique mono-alkylé lourd est formé par alkylation du benzène.

**6.** Procédé suivant la revendication 1, dans lequel le groupe alkyle de l'hydrocarbure aromatique monocyclique mono-alkylé possède 20 à 28 atomes de carbone, et dans lequel la teneur en mono-alkylat de l'hydrocarbure aromatique monocyclique mono-alkylé initial est d'au moins 80 % en poids.

**7.** Procédé suivant la revendication 1, dans lequel le catalyseur acide est une argile acide.

**8.** Procédé suivant la revendication 1, pour porter la teneur en alkylat lourd d'un mono-alkylat de benzène lourd à au moins 50 % en poids, ledit procédé comprenant la réaction du mono-alkylat de benzène lourd avec une oléfine ayant 6 à 14 atomes de carbone à une pression allant jusqu'à 10 bars et une température comprise dans l'intervalle de 100° à 225°C en présence d'un catalyseur consistant en une argile acide, dans lequel la teneur en mono-alkylat du benzène mono-alkylé initial est d'au moins 80 % en poids, et dans lequel le groupe alkyle du benzène mono-alkylé a 20 à 28 atomes de carbone.